# EUROPÄISCHE PATENTSCHRIFT

(11) **EP 0 904 285 B1**
(45) Veröffentlichungstag und Bekanntmachung des Hinweises auf die Patenterteilung: **18.09.2002**
(21) Anmeldenummer: 97917291.3
(22) Anmeldetag: 02.04.1997
(51) Int. Cl.: C07K 7/64, A61K 38/12, C07K 14/75

(54) **CYCLISCHE ADHÄSIONSINHIBITOREN**
CYCLIC ADHESION INHIBITORS
INHIBITEURS D'ADHERENCE CYCLIQUES

(30) Priorität: 06.04.1996 DE 19613933
(43) Veröffentlichungstag der Anmeldung: 31.03.1999
(73) Patentinhaber: MERCK PATENT GmbH, 64293 Darmstadt (DE)
(72) Erfinder: JONCZYK, Alfred, D-64295 Darmstadt (DE); GOODMAN, Simon, D-64286 Darmstadt (DE); DIEFENBACH, Beate, D-64283 Darmstadt (DE); KESSLER, Horst, D-85748 Garching (DE); KOPPITZ, Marcus, D-85748 Garching (DE)
(74) Vertreter: Schüttler, Reinhard, Dr.
(86) Internationale Anmeldenummer: EP9701657
(87) Internationale Veröffentlichungsnummer: WO97038009

(56) Entgegenhaltungen:
- EP-A- 0 578 083
- EP-A- 0 632 053
- DE-A- 19 534 177
- JOURNAL OF BIOLOGICAL CHEMISTRY, Bd. 269, Nr. 32, 12.August 1994, MD US, Seiten 20233-20238, XP002036951 M PFAFF ET AL.: "Selective recognition of cyclic RGD peptides of NMR defined conformation by alphaIIb-beta3, alphaV-beta3, and alpha5-betaI integrines "

## Beschreibung

Die Erfindung betrifft neue Cyclopeptide der Formel I

Cyclo-(Arg-Gly-Asp-X-Y) I,

worin
- X: Phe, homo-Phe, Phg, Phe (4-F) oder Phe (4-CI),
- Y: hPro, Ahds, Aos, Nhdg, Acha, Aib, Acpa oder Tle,
bedeuten,
wobei, sofern es sich um Reste optisch aktiver Aminosäuren oder Aminosäurederivate handelt, sowohl in D- als auch die L-Formen eingeschlossen sind, und Derivate, insbesondere Asparaginsäure β-Ester oder N-Guanidin-acyl-Derivate des Arginins, sowie deren physiologisch unbedenklichen Salze.

Ähnliche Verbindungen sind z. B. aus EP 0 406 428 und FEBS Lett. 291, 50-54 (1991) bekannt.

Andere Cyclopeptide, die die Sequenz Arg-Gly-Asp enthalten, sind offenbart in EP-A-632053, EP-A-578083 und im Journal of Biological Chemistry, Bd. 269, Nr. 32, Seiten 20233-20238.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde überraschenderweise gefunden, dass die Verbindungen der Formel I und ihre Salze sehr wertvolle Eigenschaften besitzen. Vor allem wirken sie als Integrin-Inhibitoren, wobei sie insbesondere die Wechselwirkungen der β₃- oder β₅-Integrin-Rezeptoren mit Liganden hemmen. Besondere Wirksamkeit zeigen die Verbindungen im Fall der Integrine aᵥβ₃, aᵥβ₅ und aₗₗβ₃ aber auch gegenüber αᵥβ₁-, αᵥβ₁-, αᵥβ₆- und αᵥβ₈-Rezeptoren. Diese Wirkungen können z. B. nach der Methode nachgewiesen werden, die von J.W. Smith et al. in J. Biol. Chem. 265, 12267-12271 (1990) beschrieben wird. Zusätzlich treten antiinflammatorische Effekte auf.

Die Abhängigkeit der Entstehung von Angiogenese von der Wechselwirkung zwischen vaskulären Integrinen und extrazellulären Matrixproteinen ist von P.C. Brooks, R.A. Clark und C.A. Cheresh in Science 264, 569-71 (1994) beschrieben.

Die Möglichkeit der Inhibierung dieser Wechselwirkung und die damit verbundene Einleitung von Apotosis (programmierter Zelltod) angiogener vaskulärer Zellen durch ein cyclisches Peptid ist von P.C. Brooks, A.M. Montgomery, M. Rosenfeld, R.A. Reisfeld, T.-Hu, G. Klier und D.A. Cheresh in Cell 79, 1157-64 (1994) beschrieben.

Verbindungen der Formel I, die die Wechselwirkung von Integrinrezeptoren und Liganden, wie z.B. von Fibrinogen an den Fibrinogenrezeptor (Glycoprotein IIb/IIIa) blockieren, verhindern als GPIIb/IIIa-Antagonisten die Ausbreitung von Tumorzellen durch Metastase. Dies wird durch folgende Beobachtungen belegt.

Die Verbreitung von Tumorzellen von einem lokalen Tumor in das vaskuläre System erfolgt durch die Bildung von Mikroaggregaten (Mikrothromben) durch Wechselwirkung der Tumorzellen mit Blutplättchen. Die Tumorzellen sind durch den Schutz im Mikroaggregat abgeschirmt und werden von den Zellen des Immunsystems nicht erkannt.

Die Mikroaggregate können sich an Gefäßwandungen festsetzen, wodurch ein weiteres Eindringen von Tumorzellen in das Gewebe erleichtert wird. Da die Bildung der Mikrothromben durch Fibrinogenbindung an die Fibrinogenrezeptoren auf aktivierten Blutplättchen vermittelt wird, können die GPlla/lllb-Antagonisten als wirksame Metastase-Hemmer angesehen werden.

Die Verbindungen der Formel I können ferner als antimikrobiell wirkende Substanzen bei Operationen eingesetzt werden, wo Biomaterialien, Implantate, Katheter oder Herzschrittmacher eingesetzt werden. Dabei wirken sie antiseptisch. Die Wirksamkeit der antimikrobiellen Aktivität kann durch das von P. Valentin-Weigund et al., in Infection and Immunity, 2851-2855 (1988) beschriebene Verfahren nachgewiesen werden.

Da die Verbindungen der Formel I Inhibitoren der Fibrinogenbindung und damit Liganden der Fibrinogenrezeptoren auf Blutplättchen darstellen, können sie als Diagnostika zur Detektion und Lokalisierung von Thromben im vaskulären System *in vivo* verwendet werden, sofern sie beispielsweise durch einen radioaktiven oder UV-detektierbaren Rest substituiert werden.

Die Verbindungen der Formel I können als Inhibitoren der Fibrinogenbindung auch als wirksame Hilfsmittel zum Studium des Metabolismus von Blutplättchen in unterschiedlichen Aktivierungsstadien oder von intrazellulären Signalmechanismen des Fibrinogenrezeptors verwendet werden. Die detektierbare Einheit eines einzubauenden "Labels", z.B. eine Isotopenmarkierung durch ³H, erlaubt es, nach Bindung an den Rezeptor die genannten Mechanismen zu untersuchen.

Die Verbindungen haben also die Eigenschaft, die Bindung natürlicher oder künstlicher Liganden an Integrine, speziell der Integrine αᵥβ₃, αᵥβ₅ und α_{II}β₃, aber auch von αᵥβ₆ und αᵥ₆ und αᵥβ₈ zu inhibieren.

Sie haben zudem noch den Vorteil zum Stand der Technik, dass durch α-N-Alkylierung oder α-C-Alkylierung des Y-Aminosäurerestes eine metabolische Stabilisierung und erhöhte Fettlöslichkeit erreicht wird. Durch die Reduzierung der möglichen Wasserstoffbrücken, denn N-Alkyl kann z. B. kein H-Donor für C=O sein, verbessert sich die Penetrationsfähigkeit durch Membranen, so dass eine erhöhte orale Resorbierbarkeit erhalten werden kann, zu dem kann eine gesteigerte Plasmaproteinbindung auftreten.

Die α-N-Alkylierung oder α-C-Alkylierung des Y-Aminosäurebausteines steigert die inhibitorische Potenz der Verbindungen und erhöht die Selektivität der Inhibierung in Bezug auf bestimmte Integrine.

Insbesondere durch die N-Alkyl-Gruppen kann die Selektivität beeinflusst werden.

Die Verbindungen können als Arzneimittelwirkstoffe in der Human- und Veterinärmedizin eingesetzt werden, insbesondere zur Prophylaxe und zur Behandlung von Erkrankungen des Kreislaufs, Thrombose, Herzinfarkt, Arteriosklerose, Entzündungen, Apoplexie, Angina pectoris, Tumorerkrankungen, osteolytischen Erkrankungen, insbesondere Osteoporose, Angiogenese und durch Angiogenese bedingte Erkrankungen, wie z.B. der diabetischen Retinopathie des Auges, ophthalmischen Erkrankungen, makularer Degeneration, Myopie, okularer Histoplasmose, rheumatischer Arthritis, Osteoarthritis, rubeotischem Glaukom, aber auch ulcerativen Colitis, Morbus Crohn, Multiple Sclerose, Psoriasis sowie Restenose nach Angioplastie. Femer können die Verbindungen zur Verbesserung und Unterstützung von Wundheilungsprozessen bei mikrobiellen Infekten und bei akuten Nierenversagen eingesetzt werden.

Diese Wirkungen können z. B. mit Hilfe von literaturbekannten Methoden, wie sie z. B. von P.C. Brooks et al. in Cell. 79, 1157-1164 (1994) oder Science 264, 569-571 (1994) beschrieben werden, nachgewiesen werden.

Die vor- und nachstehend aufgeführten Abkürzungen von Aminosäureresten stehen für die Reste folgender Aminosäuren:
- Abu: 4-Aminobuttersäure
- Acha: α-Aminocyclohexancarbonsäure
- Acpa: α-Aminocyclopentancarbonsäure
- Aha: 6-Aminohexansäure
- Ahds: 16-Aminohexadecansäure
- Aib: 3-Aminoisobuttersäure
- Ala: Alanin
- Aos: 8-Aminooctansäure
- Asn: Asparagin
- Asp: Asparaginsäure
- Asp(OR): Asparaginsäure (β-ester)
- Arg: Arginin
- N-Ac-Arg: N-Guanidinoacylarginin
- Cha: 3-Cyctohexylalanin
- Dab: 2,4-Diaminobuttersäure
- Dap: 2,3-Diaminopropionsäure
- Deg: Diethylglycin
- Gln: Glutamin
- Glu: Glutaminsäure
- Gly: Glycin
- hPro: Pipecolinsäure
- His: Histidin
- Ile: Isoleucin
- Leu: Leucin
- Lys: Lysin
- Nal: 3-(2-Naphthyl)-alanin
- Nhdg: N-Hexadecylglycin
- Nle: Norleucin
- Phe: Phenylalanin
- homoPhe: homo-Phenylalanin
- 4-Hal-Phe: 4-Halogen-phenylalanin
- Phg: Phenylglycin
- Pro: Prolin
- Sar: Sarcosin (N-Methylglycin)
- Tia: 3-(2-Thienyl)-alanin
- Tic: Tetrahydroisochinolin-3-carbonsäure
- Thr: Threonin
- Tle: tert. Leucin (C_{α}-tert.-butylglycin)
- Trp: Tryptophan
- Tyr: Tyrosin
- Val: Valin.

Femer bedeuten nachstehend:
- BOC: tert.-Butoxycarbonyl
- Bzl: Benzyl
- DCCI: Dicyclohexylcarbodiimid
- DMF: Dimethylformamid
- EDCI: N-Ethyl-N'-(3-dimethylaminopropyl)-carbodiimid x HCI
- Et: Ethyl
- Fmoc: 9-Fluorenylmethoxycarbonyl
- HOBt: 1-Hydroxybenzotriazol
- Me: Methyl
- Mtr: 4-Methoxy-2,3,6-trimethylphenyl-sulfonyl
- NMe: N-methylierte α-Aminogruppe
- OBut: tert.-Butylester
- OMe: Methylester
- OEt: Ethylester
- POA: Phenoxyacetyl
- TBTU: 2-(1 H-Benzotriazol-1-yl)-1,1,3,3-tetramethyluroniumtetrafluorborat
- TFA: Trifluoressigsäure.

Sofern die vorstehend genannten Aminosäuren in mehreren enantiomeren Formen auftreten können, so sind vor- und nachstehend, z. B. als Bestandteile der Verbindungen der Formel I, alle diese Formen und auch ihre Gemische (z. B. die DL-Formen) eingeschlossen. Ferner können die Aminosäuren, z. B. als Bestandteil von Verbindungen der Formel I, mit entsprechenden an sich bekannten Schutzgruppen versehen sein.

Femer werden von der Erfindung auch solche Peptide eingeschlossen, deren Aminosäurereste teilweise oder vollständig derivatisiert sind. Unter "derivatisiert" ist zu verstehen, dass auch sogenannte "Prodrugs", wie z. B. N-Guanidino-acylderivate von Arg, β-Ester von Asp, N^{ε}-Alkanoyl-, -aminoalkanoyl,-, -Mercaptoalkanoyl-Derivate des Lysins, um nur einige zu nennen, eingeschlossen werden. Außerdem können die Aminosäurereste teilweise C-alpha-alkyliert oder, z. B. für diagnostische Zwecke, isotopenmarkiert sein. Weiterhin sind solche Verbindungen der Formel I eingeschlossen, die in den Seitenketten der Bausteine X und Y zusätzlich durch Amino-, Carboxy- oder Mercaptogruppen derivatisiert sind, da derartige Derivate wichtige Ausgangsverbindungen zur Herstellung höhermolekularer Konjugate, z. B. für Immunisierungszwecke und Antikörperherstellung sind. Femer ist es möglich, funktionelle Gruppen in der Seitenkette bestimmter Aminosäurereste oder derivatisierter Aminosäurereste zur Immobilisierung der Peptide auf Polymermaterialien für die Herstellung von Affinitätschromatographiesäulen zu verwenden oder die funktionellen Gruppen zur Derivatisierung mit diagnostischen Hilfsreagenzien, wie fluoreszierenden Substituenten zu nutzen.

Gegenstand der Erfindung ist femer ein Verfahren zur Herstellung einer Verbindung der Formel I nach Anspruch 1 oder eines ihrer Salze, dadurch gekennzeichnet, dass man sie aus einem ihrer funktionellen Derivate durch Behandeln mit einem solvolysierenden oder hydrogenolysierenden Mittel in Freiheit setzt,
oder dass man ein Peptid der Formel II

H-Z-OH II

worin
- Z: -Arg-Gly-Asp-X-Y-
-Gly-Asp-X-Y-Arg-
-Asp-X-Y-Arg-Gly-
-X-Y-Arg-Gly-Asp- oder
-Y-Arg-Gly-Asp-X-
bedeutet,
oder ein reaktionsfähiges Derivat eines solchen Peptids mit einem cyclisierenden Mittel behandelt,
oder dass man ein Cyclopeptid, welches an sich der Formel I entspricht, aber über eine oder mehrere freie Aminogruppen, Säuregruppen und/oder aktivierte α-C-Atome verfügt, durch Alkylierung, Acylierung oder Veresterung derivatisiert
und/oder dass man eine basische oder saure Verbindung der Formel I durch Behandeln mit einer Säure oder Base in eines ihrer Salze überführt.

Vor- und nachstehend haben die Reste X und Y die bei den Formeln I und II angegebenen Bedeutungen, sofern nicht ausdrücklich etwas anderes angegeben ist. Die verwendeten Buchstaben für die jeweiligen Reste stehen in keinem Zusammenhang mit dem Einbuchstaben-Code für Aminosäuren.

In den vorstehenden Formeln steht Alkyl vorzugsweise für Methyl, Ethyl, Isopropt, n-Butyl, sec.-Butyl oder tert.-Butyl. Ferner bedeutet Alkyl aber auch vorzugsweise n-Pentyl, Isopentyl, Neopentyl, n-Hexyl, n-Heptyl, n-Octyl, n-Nonyl, n-Decyl oder n-Hexadecyl.

Die Gruppe X ist vorzugsweise Phe, auch bevorzugt D-Phe, aber auch Phe(4-Hal), besonders Phe(4-F) oder Phe(4-CI) sowie homo-Phe oder Phg, wobei die D-Formen auch gleichermaßen bevorzugt sind.

Y ist vorzugsweise ein hydrophober Aminosäurerest, insbesondere Gly, Ala, Val, Leu, Nle oder Ile.

Dementsprechend sind Gegenstand der Erfindung insbesondere diejenigen Verbindungen der Formel I, in denen mindestens einer der genannten Reste eine oder vorstehend angegebenen bevorzugten Bedeutungen hat.

Eine bevorzugte Gruppe von Verbindungen kann durch die Teilformel la ausgedrückt werden, die sonst der Formel I entspricht, worin jedoch
- X: D-Phe, Phe, D-homoPhe, homoPhe, D-Phg, Phg, Phe(4-F),
D-Phe(4-F), D-Phe(4-Cl) oder Phe(4-CI), und
- Y: Nle, hPro, Ahds, Aos, Nhdg, Acha, Aib, Acpa, Tle, Ala, Leu oder lle, wobei D- und L-Formen gleichermaßen bevorzugt sind,
bedeuten.

Eine weitere bevorzugte Gruppe von Verbindungen kann durch die Teilformel Ib ausgedrückt werden, die sonst der Formel I entspricht, worin jedoch
- X: D-Phe oder Phe und
- Y: Ahds, hPro, Aos, Nhdg, Acha, Aib, Acpa oder Tle, wobei D- und L-Formen gleichermaßen bevorzugt sind,
bedeuten,
und alle Aminosäurereste Arg, Gly oder Asp in der natürlichen L-Konfiguration vorliegen.

Eine weitere bevorzugte Gruppe von Verbindungen kann durch die Teilformel Ic ausgedrückt werden, die den Teilformetn la und Ib sowie der Formel I entspricht, worin jedoch nur einer der Aminosäurereste X oder X in der D-Form vorliegt, während alle anderen L-konfiguriert sind.

Ferner sind alle physiologisch verträglichen Salze der unter die Teilformeln la, lb und lc fallenden Verbindungen besonders bevorzugt.

Die Verbindungen der Formel I und auch die Ausgangsstoffe zu ihrer Herstellung werden im übrigen nach bekannten Methoden hergestellt, wie sie in der Literatur (z. B. in den Standardwerken wie Houben-Weyl, Methoden hergestellt, wie sie in der Literatur (z. B. in den Standardwerken wie Houben-Weyl, Methoden der organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von bekannten, hier nicht näher erwähnten Varianten Gebrauch machen.

Die Ausgangsstoffe können, falls erwünscht, auch in situ gebildet werden, so dass man sie aus dem Reaktionsgemisch nicht isoliert, sondern sofort weiter zu den Verbindungen der Formel I umsetzt.

Die Verbindungen der Formel I können erhalten werden, indem man sie aus ihren funktionellen Derivaten durch Solvolyse, insbesondere Hydrolyse, oder durch Hydrogenolyse in Freiheit setzt.

Bevorzugte Ausgangsstoffe für die Solvolyse bzw. Hydrogenolyse sind solche, die anstelle einer oder mehrerer freier Amino- und/oder Hydroxygruppen entsprechende geschützte Amino- und/oder Hydroxygruppen enthalten, vorzugsweise solche, die anstelle eines H-Atoms, das mit einem N-Atom verbunden ist, eine Aminoschutzgruppe tragen, z. B. solche, die der Formel I entsprechen, aber anstelle einer NH₂-Gruppe eine NHR'-Gruppe (worin R' eine Aminoschutzgruppe bedeutet, z. B. BOC oder CBZ) enthalten.

Ferner sind Ausgangsstoffe bevorzugt, die anstelle des H-Atoms einer Hydroxygruppe eine Hydroxyschutzgruppe tragen, z. B. solche, die der Formel I entsprechen, aber anstelle einer Hydroxyphenylgruppe eine R"O-phenylgruppe enthalten (worin R" eine Hydroxyschutzgruppe bedeutet).

Es können auch mehrere - gleiche oder verschiedene - geschützte Aminound/oder Hydroxygruppen im Molekül des Ausgangsstoffes vorhanden sein. Falls die vorhandenen Schutzgruppen voneinander verschieden sind, können sie in vielen Fällen selektiv abgespalten werden.

Der Ausdruck "Aminoschutzgruppe" ist allgemein bekannt und bezieht sich auf Gruppen, die geeignet sind, eine Aminogruppe vor chemischen Umsetzungen zu schützen (zu blockieren), die aber leicht entfembar sind, nachdem die gewünschte chemische Reaktion an anderen Stellen des Moleküls durchgeführt worden ist. Typisch für solche Gruppen sind insbesondere unsubstituierte oder substituierte Acyl-, Aryl-, Aralkoxymethyl- oder Aralkylgruppen. Da die Aminoschutzgruppen nach der gewünschten Reaktion (oder Reaktionsfolge) entfernt werden, ist ihre Art und Größe im übrigen nicht kritisch; bevorzugt werden jedoch solche mit 1-20, insbesondere 1-8 C-Atomen. Der Ausdruck "Acylgruppe" ist im Zusammenhang mit dem vorliegenden Verfahren in weitestem Sinne aufzufassen. Er umschließt von aliphatischen, araliphatischen, aromatischen oder heterocyclischen Carbonsäuren oder Sulfonsäuren abgeleitete Acylgruppen sowie insbesondere Alkoxycarbonyl-, Aryloxycarbonyl- und vor allem Aralkoxycarbonylgruppen. Beispiele für derartige Acylgruppen sind Alkanoyl wie Acetyl, Propionyl, Butyryl; Aralkanoyl wie Phenylacetyl; Aroyl wie Benzoyl oder Toluoyl; Aryloxyalkanoyl wie POA; Alkoxycarbonyl wie Methoxycarbonyl, Ethoxycarbonyl, 2,2,2-Trichlorethoxycarbonyl, BOC, 2-Jod-ethoxycarbonyl; Aralkyloxycarbonyl wie CBZ ("Carbobenzoxy"), 4-Methoxybenzyloxycarbonyl, FMOC; Arylsulfonyl wie Mtr. Bevorzugte Aminoschutzgruppen sind BOC und Mtr, femer CBZ, Fmoc, Benzyl und Acetyl.

Der Ausdruck "Hydroxyschutzgruppe" ist ebenfalls allgemein bekannt und bezieht sich auf Gruppen die geeignet sind, eine Hydroxygruppe vor chemischen Umsetzungen zu schützen, die aber leicht entfembar sind, nachdem die gewünschte chemische Reaktion an anderen Stellen des Moleküls durchgeführt worden ist. Typisch für solche Gruppen sind die oben genannten unsubstituierten oder substituierten Aryl-, Aralkyl- oder Acylgruppen, ferner auch Alkylgruppen. Die Natur und Größe der Hydroxyschutzgruppen ist nicht kritisch, da sie nach der gewünschten chemischen Reaktion oder Reaktionsfolge wieder entfernt werden; bevorzugt sind Gruppen mit 1-20, insbesondere 1-10 C-Atomen. Beispiele für Hydroxyschutzgruppen sind u.a. Benzyl, p-Nitrobenzoyl, p-Toluolsulfonyl, tert.Butyl und Acetyl, wobei Benzyl und tert.Butyl besonders bevorzugt sind. Die COOH-Gruppen in Asparaginsäure und Glutaminsäure werden bevorzugt in Form ihrer tert.-Butlester geschützt (z. B. Asp(OBut)).

Die als Ausgangsstoffe zu verwendenden funktionellen Derivate der Verbindungen der Formel I können nach üblichen Methoden der Aminosäure- und Peptidsynthese hergestellt werden, wie sie z. B. in den genannten Standardwerken und Patentanmeldungen beschrieben sind, z. B. auch nach der Festphasenmethode nach Merrifield (B.F. Gysin u. R.B. Merrifield, J. Am. Chem. Soc. 94, 3102 ff. (1972)).

Das In-Freiheit-Setzen der Verbindungen der Formel I aus ihren funktionellen Derivaten gelingt- je nach der benutzten Schutzgruppe - z. B. mit starken Säuren, zweckmäßig mit TFA oder Perchlorsäure, aber auch mit anderen starken anorganischen Säuren wie Salzsäure oder Schwefelsäure, starken organischen Carbonsäuren wie Trichloressigsäure oder Sulfonsäuren wie Benzol- oder p-Toluolsulfonsäure, Die Anwesenheit eines zusätzlichen inerten Lösungsmitels ist möglich, aber nicht immer erforderlich. Als inerte Lösungsmittel eignen sich vorzugsweise organische, beispielsweise Carbonsäuren wie Essigsäure, Ether wie Tetrahydrofuran oder Dioxan, Amide wie DMF, halogenierte Kohlenwasserstoffe wie Dichlormethan, femer auch Alkohole wie Methanol, Ethanol oder Isopropanol sowie Wasser. Femer kommen Gemische der vorgenannten Lösungsmittel in Frage. TFA wird vorzugsweise im Überschuss ohne Zusatz eines weiteren Lösungsmittels verwendet, Perchlorsäure in Form eines Gemisches aus Essigsäure oder 70%iger Perchlorsäure im Verhältnis 9 : 1. Die Reaktionstemperaturen für die Spaltung liegen zweckmäßig zwischen etwa 0 und etwa 50°, vorzugsweise arbeitet man zwischen 15 und 30° (Raumtemperatur).

Die Gruppen BOC, OBut und Mtr können z. B. bevorzugt mit TFA in Dichlormethan oder mit etwa 3 bis 5 n HCI in Dioxan bei 15-30° abgespalten werden, die FMOC-Gruppe mit einer etwa 5- bis 50%igen Lösung von Dimethylamin, Diethylamin oder Piperidin in DMF bei 15-30°.

Hydrogenolytisch entfembare Schutzgruppen (z. B. CBZ oder Benzyl) können z. B. durch Behandeln mit Wasserstoff in Gegenwart eines Katalysators (z. B. eines Edelmetallkatalysators wie Palladium, zweckmäßig auf einem Träger wie Kohle) abgespalten werden. Als Lösungsmittel eignen sich dabei die oben angegebenen, insbesondere z. B. Alkohole wie Methanol oder Ethanol oder Amide wie DMF. Die Hydrogenolyse wird in der Regel bei Temperaturen zwischen etwa 0 und 100° und Drucken zwischen etwa 1 und 200 bar, bevorzugt bei 20-30° und 1-10 bar durchgeführt. Eine Hydrogenolyse der CBZ-Gruppe gelingt z. B. gut an 5 bis 10%igem Pd-C in Methanol oder mit Ammoniumformiat (anstelle von H₂) an Pd-C in Methanol/DMF bei 20-30°.

Verbindungen der Formel I können auch durch Cyclisierung von Verbindungen der Formel II unter den Bedingungen einer Peptidsynthese erhalten werden. Dabei arbeitet man zweckmäßig nach üblichen Methoden der Peptid-Synthese, wie sie z. B. in Houben-Weyl, 1.c., Band 15/II, Seiten 1 bis 806 (1974) beschrieben sind.

Die Reaktion gelingt vorzugsweise in Gegenwart eines Dehydratisierungsmittels, z. B. eines Carbodiimids wie DCCI oder EDCI, femer Propanphosphonsäureanhydrid (vgl. Angew. Chem. 92, 129 (1980)), Diphenylphosphorylazid oder 2-Ethoxy-N-ethoxycarbonyl-1,2-dihydrochinolin, in einem inerten Lösungsmittel, z. B. einem halogenierten Kohlenwasserstoff wie Dichlormethan, einem Ether wie Tetrahydrofuran oder Dioxan, einem Amid wie DMF oder Dimethylacetamid, einem Nitril wie Acetonitril, oder in Gemischen dieser Lösungsmittel, bei Temperaturen zwischen etwa -10 und 40, vorzugsweise zwischen 0 und 30°. Um die intramolekulare Cyclisierung vor der intermolekularen Peptid-Bindung zu fördern, ist es zweckmäßig, in verdünnten Lösungen zu arbeiten (Verdünnungsprinzip).

Anstelle von II können auch geeignete reaktionsfähige Derivate dieser Stoffe in die Reaktion eingesetzt werden, z. B. solche, in denen reaktive Gruppen intermediär durch Schutzgruppen blockiert sind. Die Aminosäurederivate II können z. B. in Form ihrer aktivierten Ester verwendet werden, die zweckmäßig in situ gebildet werden, z. B. durch Zusatz von HOBt oder N-Hydroxysuccinimid.

Die Ausgangsstoffe der Formet II sind in der Regel neu. Sie können nach bekannten Methoden, z. B. den oben angegebenen Methoden der Peptidsynthese und der Abspaltung von Schutzgruppen, hergestellt werden.

In der Regel synthetisiert man zunächst geschützte Pentapeptidester der Formel R'-Z-OR", z. B. BOC-Z-OMe oder BOC-Z-OEt, die zunächst zu Säuren der Formel R'-Z-OH, z. B. BOC-Z-OH verseift werden; aus diesen wird die Schutzgruppe R' abgespalten, wodurch man die freien Peptide der Formel H-Z-OH (II) erhält.

Die Derivatisierung eines Cyclopeptides, welches an sich einer Verbindung der Formel I entspricht, erfolgt ebenfalls über an sich bekannte Methoden, wie sie für die Alkylierung von Aminen, die Veresterung von Carbonsäuren oder die nucleophile Substitution an aliphatischen C-Atomen bekannt und in jedem Lehrbuch der Organischen Chemie, z. B. J. March, Adv. Org. Chem., John Wiley & Sons N.Y. (1985), beschrieben sind.

Eine Base der Formel I kann mit einer Säure in das zugehörige Säureadditionssalz übergeführt werden. Für diese Umsetzung kommen insbesondere Säuren in Frage, die physiologisch unbedenkliche Salze liefern. So können anorganische Säuren verwendet werden, z. B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, femer organische Säuren, insbesondere aliphatische, alicyclische, araliphatische, aromatische oder heterocyclische ein- oder mehrbasige Carbon-, Sulfon- oder Schwefelsäuren, z. B. Ameisensäure, Essigsäure, Propionsäure, Pivalinsäure, Diethylessigsäure, Malonsäure, Bemsteinsäure, Pimelinsäure, Fumarsäure, Maleinsäure, Milchsäure, Weinsäure, Äpfelsäure, Benzoesäure, Salicylsäure, 2- oder 3-Phenylpropionsäure, Citronensäure, Gluconsäure, Ascorbinsäure, Nicotinsäure, Isonicotinsäure, Methan - oder Ethansulfonsäure, Ethandisulfonsäure, 2-Hydroxyethansulfonsäure, Benzolsulfonsäure, p-Toluolsulfonsäure, Naphthalin-mono- und -diusulfonsäuren, Laurylschwefelsäure. Salze mit physiologisch nicht unbedenklichen Säuren, z. B. Pikrate, können zur Isolierung und/oder Aufreinigung der Verbindungen der Formel I verwendet werden.

Andererseits kann eine Säure der Formel I durch Umsetzung mit einer Base in eines ihrer physiologisch unbedenklichen Metall- oder Ammoniumsalze übergeführt werden. Als Salze kommen dabei insbesondere die Natrium-, Kalium-, Magnesium-, Calcium- und Ammoniumsalze in Betracht, femer substituierte Ammoniumsalze, z. B. die Dimethyl-, Diethyl- oder Diisopropylammoniumsalze, Monoethanol-, Diethanol- oder Triethanolammoniumsalze, Cyclohexyl-, Dicyclohexylammoniumsalze, Dibenzylethylendiammoniumsalze, weiterhin z. B. Salze mit N-Methyl-D-glucamin oder mit Arginin oder Lysin.

Die neuen Verbindungen der Formel I und ihre physiologisch unbedenklichen Salze können zur Herstellung pharmazeutischer Präparate verwendet werden, indem man sie zusammen mit mindestens einem Träger- oder Hilfsstoff und, falls erwünscht, zusammen mit einem oder mehreren weiteren Wirkstoff(en) in eine geeignete Dosierungsform bringt. Die so erhaltenen Zubereitungen können als Arzneimittel in der Humanoder Veterinärmedizin eingesetzt werden. Als Trägersubstanzen kommen organische oder anorganische Stoffe in Frage, die sich für die enterale (z. B. orale oder rektale), parenterale (z. B. intravenöse Injektion) oder lokale (z. B. topische, dermale, ophthalmische oder nasale) Applikation oder für eine Applikation in Form eines Inhalations-Sprays eignen und mit den neuen Verbindungen nicht reagieren, beispielsweise Wasser oder wässerige isotonische Kochsalzlösung, niedere Alkohole, pflanzliche Öle, Benzylalkohole, Polyethylenglykole, Glycerintriacetat und andere Fettsäureglyceride, Gelatine, Sojalecithin, Kohlehydrate wie Lactose oder Stärke, Magnesiumstearat, Talk, Cellulose, Vaseline. Zur oralen Anwendung dienen insbesondere Tabletten, Dragees, Kapseln, Sirupe, Säfte oder Tropfen; von Interesse sind speziell Lacktabletten und Kapseln mit magensaftresistenten Überzügen bzw. Kapselhüllen. Zur rektalen Anwendung dienen Suppositorien, zur parenteralen Applikation Lösungen, vorzugsweise ölige oder wässerige Lösungen, femer Suspensionen, Emulsionen oder Implantate. Zur topischen Anwendung eignen sich z. B. Lösungen, die in Form von Augentropfen verwendet werden können, ferner z.B. Suspensionen, Emulsionen, Cremes, Salben oder Komprimate. Für die Applikation als Inhalations-Spray können Sprays verwendet werden, die den Wirkstoff entweder gelöst oder suspendiert in einem Treibgas oder Treibgasgemisch (z. B. CO₂ oder Fluorchlorkohlenwasserstoffersatzstoffe) enthalten. Zweckmäßig verwendet man den Wirkstoff dabei in mikronisierter Form, wobei ein oder mehrere zusätzliche physiologisch verträgliche Lösungsmittel zugegeben sein können, z. B. Ethanol. Inhalationslösungen können mit Hilfe üblicher Inhalatoren verabfolgt werden. Die neuen Verbindungen können auch lyophilisiert und die erhaltenen Lyophilisate z. B. zur Herstellung von Injektionspräparaten verwendet werden. Die Injektionen können dabei als Bolus oder als kontinuierliche Infusion (z. B. intravenös, intramusculär, subcutan oder intrathecal) gegeben werden. Die angegebenen Zubereitungen können sterilisiert sein und/oder Hilfsstoffe wie Konservierungs-, Stabilisierungsund/oder Netzmittel, Emulgatoren, Salze zur Beeinflussung des osmotischen Druckes, Puffersubstanzen, Farb- und/oder Aromastoffre enthalten. Sie können, falls erwünscht, auch einen oder mehrere weitere Wirkstoffe enthalten, z. B. auch ein oder mehrere Vitamine.

Die erfindungsgemäßen Substanzen können in der Regel in Analogie zu anderen bekannten, im Handel befindlichen Peptiden, insbesondere aber in Analogie zu den in US 4,472,305 beschriebenen Verbindungen verabreicht werden, vorzugsweise in Dosierungen zwischen etwa 0,05 und 500, insbesondere zwischen 0,5 und 100 mg pro Dosierungseinheit. Die tägliche Dosierung liegt vorzugsweise zwischen etwa 0,01 und 2 mg/kg Körpergewicht. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die parenterale Applikation ist bevorzugt.

Femer können die neuen Verbindungen der Formel I als Integrinliganden zur Herstellung von Säulen für die Affinitätschromatographie zur Reindarstellung von Integrinen verwendet werden.

Der Ligand, d. h. ein Peptidderivat der Formel I, wird dabei über Ankerfunktionen an einen polymeren Träger kovalent gekuppelt.

Als polymere Trägermaterialien eignen sich die an sich in der Peptidchemie bekannten polymeren festen Phasen mit vorzugsweise hydrophilen Eigenschaften, beispielsweise quervemetzte Polyzucker, wie Zellulose, Sepharose oder Sephadex®, Acrylamide, Polymer auf Polyethylenglykolbasis oder Tentakelpolymere®.

Als Ankerfunktionen, die mit den polymeren Trägern verknüpft sind, eignen sich vorzugsweise lineare Alkylenketten mit 2-12 C-Atomen, die mit einem Ende direkt an das Polymer gebunden sind und am andem Ende eine funktionelle Gruppe, wie z. B. Hydroxy, Amino, Mercapto, Maleinimido oder -COOH aufweisen und dazu geeignet sind, mit dem C- oder N-terminalen Abschnitt des jeweiligen Peptids verknüpft zu werden.

Dabei ist es möglich, dass das Peptid direkt oder ebenfalls über eine zweite Ankerfunktion mit dem Anker des Polymers verbunden ist. Femer ist es möglich, dass Peptide, die Aminosäurereste mit funktionalisierten Seitenketten enthalten, über diese mit der Ankerfunktion des Polymers verbunden werden.

Darüber hinaus können bestimmte Aminosäurerest, die Bestandteil der Peptide der Formel I sind, in ihren Seitenketten derart modifiziert werden, so dass sie zur Verankerung über z. B. SH-, OH-, NH₂- oder COOH-Gruppen mit dem Anker des Polymers zur Verfügung stehen.

Möglich sind hierbei ungewöhnliche Aminosäuren, wie z.B. Phenylalaninderivate, die in 4-Positionen des Phenylrings eine Mercapto-, Hydroxy-, Amino- oder Carboxyalkylkette tragen, wobei die funktionelle Gruppe sich am Ende der Kette befindet.

Beispiele für Aminosäurereste, deren Seitenkettedirekt als Ankerfunktion dienen kann, sind z. B. Lys, Arg, Asp, Asn, Glu, GIn, Ser, Thr, Cys oder Tyr.

Beispiele für N-terminale Anker sind Reste wie z. B. -CO-CₙH₂ₙ-NH₂, -CO-CₙH₂ₙ-OH, -CO-CₙH₂ₙ-SH oder -CO-Cₙ-H₂ₙ-COOH mit n = 2-12, wobei die Länge der Alkylenkette nicht kritisch ist und diese gegebenenfalls auch z. B. durch entsprechende Aryl- oder Alkylarylreste ersetzt werden kann. C-terminale Anker können beispielsweise -O-CₙH₂ₙ-SH-, -O-CₙH₂ₙ-OH, -O-CₙH₂ₙNH₂, -O-CₙH₂ₙ-COOH, -NH-CₙH₂ₙ-SH, -NH-CₙH₂ₙ-OH, -NH-CₙH₂ₙ₋NH₂ oder -NH-CₙH₂ₙ-COO sein, wobei für n sowie die Alkylenkette das bereis im vorhergehenden Abschnitt Gesagte gilt.

Die N- und C-terminalen Anker können auch als Ankerbausteine für eine bereits funktionalisierte Seitenkette eines Aminosäurerests dienen. Es kommen hier beispielsweise Aminosäurereste wie Lys (CO-C₅H₁₀-NH₂), Asp(NH-C₃H₆-COOH) oder Cys(C₃H₆-NH₂) in Frage, wobei der Anker immer an die funktionelle Gruppe der Seitenkette gefunden ist.

Die Herstellung der Materialien für die Affinitätschromatographie zur Integrinreinigung erfolgt unter Bedingungen wie sie für die Kondensation von Aminosäuren üblich und an sich bekannt sind und bereits im Abschnitt zur Herstellung der Verbindungen der Formel I geschildert wurden.

Neben der Verwendung der Cyclopeptide zur Immobilisierung auf Polymermaterialien für die Herstellung von Affinitätschromatographiesäulen ist es möglich, die Verbindungen mit ihren funktionalisierten Seitenketten zur weiteren Derivatisierung mit diagnostischen Hilfsreagenzien, wie z. B. fluoreszierenden Substituenten, zu nutzen.

Es ist femer möglich, in den Seitenketten der Reste X und Y zusätzlich funktionelle Gruppen wie Amino-, Mercapto- oder Carboxygruppen einzuführen, über die dann Konjugate mit Proteinen oder anderen hochmolekularen Substanzen, wie z. B. für Immunisierungszwecke und/oder Antikörpererzeugung, hergestellt werden können.

Vor- und nachstehend sind alle Temperaturen in °C angegeben. In den nachfolgenden Beispielen bedeutet "übliche Aufarbeitung": Man gibt, falls erforderlich, Wasser dazu, neutralisiert, extrahiert mit Ether oder Dichlormethan, trennt ab, trocknet die organische Phase über Natriumsulfat, filtriert, dampft ein und reinigt durch Chromatographie an Kieselgel und/oder Kristallisation. RZ = Retentio'nszeit (Minuten). Die analytische Untersuchung erfolgte durch HPLC an LiChrosorb® RP select B (7 µm)-250 x 4 mm-Säule, Eluent A: 0,3 % TFA in Wasser, Eluent B: 0,3 % TFA in 2-Propanol/Wasser (8:2) Gradient 1-99 % B in 50 Min. bei 1 ml/Min. Fluss und Detektion bei 215 nm. M⁺ = Molekular-Peak im Massenspektrum, erhalten nach der "Fast Atom Brombardment"-Methode (FAB).

### Beispiel 1

Eine Lösung von 1,1 g H-Arg(Mtr)-Gly-Asp(OBut)-D-Phe-hPro-ONa [z. B. erhältlich aus Fmoc-Nme-Arg(Mtr)-Gly-Asp(OBut)-D-Phe-hPro-O-Wang, wobei -O-Wang den bei den modifizierten Merrifield-Techniken verwendeten Rest eines 4-Oxymethyl-phenoxymethyl-polystyrolharzes bedeutet, durch Abspaltung der Fmoc-Gruppe mit Piperidin/DMF und Abspaltung des Harzes mit TFA/CH₂Cl₂ (1:1)] in 15 ml DMF wird mit 85 ml Dichlormethan verdünnt und mit 50 mg NaHCO₃ versetzt. Nach Kühlung in einer Trockeneis/Aceton-Mischung werden 40 µl Diphenylphosphorylazid zugegeben. Nach 16 Stunden Stehen bei Raumtemperatur engt man die Lösung ein. Das Konzentrat wird gelfiltriert (Sephadex G10-Säule in Isopropanol/Wasser 8:2) und dann wie üblich mittels HPLC gereinigt. Man erhält nach Behandlung mit TFA/H₂O (98:2) Cyclo-(Arg-Gly-Asp-D-PhehPro); RZ = 18,5; FAB-MS (M+H): 587

Analog erhält man durch Cyclisierung der entsprechenden linearen Peptide und Abspaltung der Schutzgruppen:
Cyclo-(Arg-Gly-Asp-Phe-Ahds); Rz = 35,1; FAB-MS (M+H): 730;
Cyclo-(Arg-Gly-Asp-DPhe-Ahds); RZ = 35,4; FAB-MS (M+H): 730;
Cyclo-(Arg-Gly-Asp-Phe-DAhds); RZ = 35,7; FAB-MS (M+H): 730;

Cyclo-(Arg-Gly-Asp-DPhe-Aos);
Cyclo-(Arg-Gly-Asp-DPhe-DAos);
Cyclo-(Arg-Gly-Asp-Phe-DAos);

Cyclo-(Arg-Gly-Asp-DPhe-Nhdg); RZ = 36,7; FAB-MS (M+H): 758;
Cyclo-(Arg-Gly-Asp-Phe-Nhdg); RZ = 36,5; FAB-MS (M+H): 758;
Cyclo-(Arg-Gly-Asp-DPhe-DNhdg); FAB-MS (M+H): 758;
Cyclo-(Arg-Gly-Asp-Phe-DNhdg); FAB-MS (M+H): 758;
Cyclo-(Arg-Gly-Asp-DPhg-Nhdg);
Cyclo-(Arg-Gly-Asp-Phg-Nhdg);
Cyclo-(Arg-Gly-Asp-DPhg-DNhdg);
Cyclo-(Arg-Gly-Asp-Phg-DNhdg);

Cydo-(Arg-Gly-Asp-DPhe-Acha); RZ = 25,2; FAB-MS (M+H): 601;
Cyclo-(Arg-Gly-Asp-Phe-Acha); FAB-MS (M+H): 601;
Cyclo-(Arg-Gly-Asp-DPhe-DAcha); FAB-MS (M+H): 601;
Cyclo-(Arg-Gly-Asp-Phe-DAcha); FAB-MS (M+H): 601;

Cyclo-(Arg-Gly-Asp-DPhe-Aib); FAB-MS (M+H): 575;
Cyclo-(Arg-Gly-Asp-Phe-aib); RZ = 36,5; FAB-MS (M+H): 575;
Cyclo-(Arg-Gly-AspDPhe-DAib); FAB-MS (M+H): 575;
Cyclo-(Arg-Gly-Asp-Phe-DAib); FAB-MS (M+H): 575;

Cyclo-(Arg-Gly-Asp-DPhe-Acpa); RZ = 17,1; FAB-MS (M+H): 587;
Cyclo-(Arg-Gly-Asp-Phe-Acpa); FAB-MS (M+H): 587;
Cyclo-(Arg-Gly-Asp-DPhe-DAcpa); FAB-MS (M+H): 587;
Cyclo-(Arg-Gly-Asp-Phe-Dcpa); FAB-MS (M+H): 587;

Cyclo-(Arg-Gly-Asp-DPhe-Tle); RZ = 19,1; FAB-MS (M+H): 589;
Cyclo-(Arg-Gly-Asp-Phe-Tle); FAB-MS (M+H): 589;
Cyclo-(Arg-Gly-Asp-DPhe-DTle); FAB-MS (M+H): 589;
Cyclo-(Arg-Gly-Asp-Phe-DTIe); FAB-MS (M+H): 589;

Cyclo-(Arg-Gly-Asp-DPhe(4-Cl)-Tle); RZ = 23,2; FAB-MS (M+H): 623;
Cyclo-(Arg-Gly-Asp-Phe(4-Cl)-Tle); FAB-MS (M+H): 623;
Cyclo-(Arg-Gly-Asp-DPhe(4-Cl)-DTle); FAB-MS (M+H): 623;
Cydo-(Arg-Gly-Asp-Phe(4-Cl)-DTle); FAB-MS (M+H): 623;

Cyclo-(Arg-Gly-Asp-DPhe(4-F)-Tle); RZ = 20,2; FAB-MS (M+H): 607;
Cyclo-(Arg-Gly-Asp-Phe(4-F)-Tle); FAB-MS (M+H): 607;
Cyclo-(Arg-Gly-Asp-DPhe(4-F)-DTle); FAB-MS (M+H): 607;
Cyclo-(Arg-Gly-Asp-Phe(4-f)-DTle), FAB-MS (M+H): 607.

### Beispiel 2

Eine Lösung von 0,28 g Cyclo-(Arg(Mtr)-Gly-Asp-DPhe-DhPro [erhältlich durch Cyclisierung gemäß Bsp 1] in 8,4 ml TFA, 1,7 ml Dichtomnethan und 0,9 ml Thiophenol wird 4 Stunden bei Raumtemperatur stehen gelassen, anschließend eingeengt und nach Verdünnen mit Wasser gefriergetrocknet. Gelfiltration an Sephadex G 10 (Essigsäure/Wasser 1:1) und anschließende Reinigung durch präparative HPLC unter den angegebenen Bedingungen liefern Cyclo-(Arg-Gly-Asp-DPhe-DhPro);
FAB-MS (M+H): 587.

Analog erhält man:
aus Cyclo-(Arg(Mtr)-Gly-Asp-Phe-DhPro):
   Cyclo-(Arg-Gly-Asp-Phe-DhPro); FAB-MS (M+H): 587;
aus Cyclo-(Arg(Mtr)-Gly-Asp(OBut)-DPhg-Tle):
   Cyclo-(D-Arg-NmeGly-Asp-DPhg-Tle);
aus Cyclo-(Arg(Mtr)-Gly-Asp(OEt)-DPhg-hPro):
   Cyclo-(Arg-Gly-Asp-DPhg-hPro);
aus Cyclo-(Arg(Mtr)-Gly-Asp-Phg-DAhds):
   Cyclo-(Arg-Gly-Asp-Phg-DAhds);
aus Cyclo-(Arg(Mtr)-Gly-Asp-DPhg-Acpa):
   Cyclo-(Arg-Gly-Asp-DPhg-Acpa);
aus Cyclo-(Arg(Mtr)-Gly-Asp-DPhg-Aos):
   Cyclo-(Arg-Gly-Asp-DPhg-Aos).

### Beispiel 3

80 mg Cyclo-(Arg-Gly-Asp-DPhe-hPro) [erhältlich nach Bsp. 1] werden fünf- bis sechsmal in 0,01 m HCI gelöst und nach jeden Lösevorgang gefriergetrocknet. Anschließende Reinigung durch HPLC liefert Cyclo-(Arg-Gly-Asp-DPhe-hPro) x HCI.

Analog erhält man
aus Cyclo-(Arg-Gly-Asp-DPhe-Nle):
   Cyclo-(Arg-Gly-Asp-DPhe-Nle): x HCI;
aus Cyclo-(Arg-Gly-Asp-DPhe-Ahds):
   Cyclo-(Arg-Gly-Asp-DPhe-Ahds) x HCI;
aus Cyclo-(Arg-Gly-Asp-Phe-Ahds):
   Cyclo-(Arg-Gly-Asp-Phe-Ahds) x HCI.

### Beispiel 4

Zur Herstellung von Affinitätsphasen suspendiert man 0,9 g N-Maleinimido-(CH₂)₅-CO-NH-(CH₂)₃-Polymer [erhältlich durch Kondenstion von N-Maleinimido-(CH₂)₅-COOH mit H₂N-(CH₂)₃-Polymer] in 10 ml 0,1 M Natriumphosphatpuffer bei pH 7 und fügt bei 4° ein Äquivalent Cyclo-(Arg-Gly-Asp-DPhe(4-N-CO(CH₂)₂SH)-hPro) [erhältlich durch Cyclisierung von H-DPhe(4-NH-BOC)-hPro-Arg(Mtr)-Gly-Asp-OH, Abspaltung der Schutzgruppen und Acylierung mit z. B. Cl-CO(CH₂)₂SH] hinzu. Man rührt 4 Stunden bei gleichzeitiger Erwärmung der Reaktionsmischung auf Raumtemperatur, filtriert den festen Rückstand ab und wäscht zweimal mit je 10 ml Pufferlösung (pH 7) und anschließend dreimal mit je 10 ml Wasser. Man erhält Cyclo-(Arg-Gly-Asp-DPhe(4-N-CO(CH₂)₂S-3-(Nmaleinimido-(CH₂)₅-CONH-(CH₂)₃-Polymer)-hPro)).

### Beispiel 5

Analog Beispiel 4 erhält man durch Kondensation von Polymer-O-(CH₂)₃-NH₂ [im Handel erhältlich] und Cyclo-(Arg-Gly-Asp-DPhe[4-N-O(CH₂)₄-COOH)-hPro) [erhältlich durch Kondensation von Adipinsäure mit Cyclo(Arg(Mtr)-Gly-Asp-DPhe(4-NH-BOC)-hPro) unter den in Bsp. 4 genannten Bedingungen] die folgende polymere Phase: Cyclo-(Arg-Gly-Asp-DPhe(4-N-CO-(CH₂)₄-CO-NH-(CH₂)₃-O-Polymer)-hPro).

Die nachstehenden Beispiele betreffen pharmazeutische Zubereitungen.

### Beispiel A: Injektionsgläser

Eine Lösung von 100 g eines Cyclopeptides der Formel I und 5 g Dinatriumhydrogenphosphat in 3 l zweifach destilliertem Wasser wird mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel B: Suppositorien

Man schmilzt ein Gemisch von 20 g Wirkstoff der Formel I mit 100 g Sojalecithin und 1400 g Kakaobutter, gießt in Formen und lässt erkalten. Jedes Suppositorium enthält 20 mg Wirkstoff.

### Beispiel C: Lösung

Man bereitet eine Lösung aus 1 g Wirkstoff der Formel I, 9,38 g NaH₂PO₄ x 2 H₂O, 28,48 g Na₂HPO₄ x 12 H₂O und 0,1 g Benzalkoniumchlorid in 940 ml zweifach destilliertem Wasser. Man stellt auf pH 6,8 ein, füllt auf 1 I auf und sterilisiert durch Bestrahlung. Diese Lösung kann in Form von Augentropfen verwendet werden.

### Beispiel D: Salbe

Man mischt 500 mg Wirkstoff der Formel I mit 99,5 g Vaseline unter aseptischen Bedingungen.

### Beispiel E: Tabletten

Ein Gemisch von 100 g eines Cyclopeptids der Formel I, 1 kg Lactose, 600 g mikrokristalliner Cellulose, 600 g Maisstärke, 100 g Polyvinylpyrrolidon, 80 g Talk und 10 g Magnesiumstearat wird in üblicher Weise zu Tabletten gepresst, so dass jede Tablette 10 g Wirkstoff enthält.

### Beispiel F: Dragees

Man presst Tabletten wie in Beispiel E angegeben und überzieht sie anschließend in üblicher Weise mit einem Überzug aus Saccharose, Maisstärke, Talk, Tragant und Farbstoff.

### Beispiel G: Kapseln

In üblicher Weise werden Hartgelatinekapseln mit einem Wirkstoff der Formel I gefüllt, so dass jede Kapsel 5 mg Wirkstoff enthält.

### Beispiel H: Inhalationsspray

Man löst 14 g Wirkstoff der Formel I in 10 I isotonischer NaCl-Lösung und füllt die Lösung in handelsübliche Sprühgefäße mit Pump-Mechanismus. Die Lösung kann in Mund oder Nase gesprüht werden. Ein Sprühstoß (etwa 0,1 ml) entspricht einer Dosis von etwa 0,14 mg

## Patentansprüche

1. Verbindungen der Formel I
Cyclo-(Arg-Gly-Asp-X-Y) I
worin
X Phe, homoPhe, Phg, Phe(4-F) oder Phe(4-Cl),
Y hPro, Ahds, Aos, Nhdg, Acha, Aib, Acpa oder Tle,
bedeuten,
wobei, sofern es sich um Reste optisch aktiver Aminosäuren und Aminosäurederivate handelt, sowohl die D- als auch die L-Formen eingeschlossen sind, und Derivate, insbesondere Asparaginsäure-β-ester oder N-Guanidin-acyl-Derivate des Arginins, sowie deren physiologische unbedenklichen Salze.

2. Ein Enantiomer oder ein Diastereomer einer Verbindung der Formel I gemäß Anspruch 1.

3. (a) Cyclo-(Arg-Gly-Asp-D-Phe-Nle);
(b) Cyclo-(Arg-Gly-Asp-DPhe-hPro);
(c) Cyclo-(Arg-Gly-Asp-DPhe-Tle);
(d) Cyclo-(Arg-Gly-Asp-Phe-DAhds);
(e) Cyclo-(Arg-Gly-Asp-Phe-Nhdg);
(f) Cyclo-(Arg-Gly-Asp-DPhe-Acha);
(g) Cyclo-(Arg-Gly-Asp-DPhe(4-Cl)-Tle);
(h) Cyclo-(Arg-Gly-Asp-DPhe(4-F)-Tle)
gemäß Anspruch 1 sowie deren physiologisch unbedenklichen Salze.

4. Verfahren zur Herstellung einer Verbindung der Formel I nach Anspruch 1 oder eines ihrer Salze, **dadurch gekennzeichnet, dass** man sie aus einem ihrer funktionellen Derivate durch Behandeln mit einem solvolysierenden oder hydrogenolysierenden Mittel in Freiheit setzt,
oder dass man ein Peptid der Formel II
H-Z-OH II
worin
Z -Arg-Gly-Asp-X-Y-
-Gly-Asp-X-Y-Arg-
-Asp-X-Y-Arg-Gly-
-X-Y-Arg-Gly-Asp- oder
-Y-Arg-Gl-AspX-
bedeutet,
oder ein reaktionsfähiges Derivat eines solchen Peptids mit einem cyclisierenden Mittel behandelt,
oder dass man ein Cyclopeptid, welches an sich der Formel I entspricht, aber auch über eine oder mehrer freie Aminogruppen, Säuregruppen und/oder aktivierte α-C-Atome verfügt, durch Alkylierung, Acylierung oder Veresterung derivatisiert,
und/oder dass man eine basische oder saure Verbindung der Formel I durch Behandeln mit einer Säure oder Base in eines ihrer Salze überführt.

5. Verfahren zur Herstellung pharmazeutischer Zubereitungen, **dadurch gekennzeichnet, dass** man eine Verbindung der Formel I nach Anspruch 1 und/oder eines ihrer physiologisch unbedenklichen Salze zusammen mit mindestens einem festen, flüssigen oder halbflüssigen Träger- oder Hilfsstoff in eine geeignete Dosierungsform bringt.

6. Pharmazeutische Zubereitung, **gekennzeichnet durch** einen Gehalt an mindestens einer Verbindung der allgemeinen Formel I nach Anspruch 1 und/oder einem ihrer physiologisch unbedenklichen Salze.

7. Verwendung von Verbindungen der Formel I nach Anspruch 1 oder von deren physiologisch unbedenklichen Salzen zur Herstellung eines Arzneimittels zur Bekämpfung von Krankheiten.

8. Verwendung von Verbindungen der Formel I nach Anspruch 1 zur Herstellung von immobilisierten Liganden für Affinitätssäulenchromatographie.

9. Verwendung von Verbindungen der Formel I nach Anspruch 1 zur Reinigung von Integrinen durch Affinitätschromatographie.

## Claims

1. Compounds of the formula I
cyclo- (Arg-Gly-Asp-X-Y) I,
in which
X is Phe, homoPhe, Phg, Phe(4-F) or Phe(4-Cl),
Y is hPro, Ahds, Aos, Nhdg, Acha, Aib, Acpa or Tle,
and, where residues of optically active amino acids and amino acid derivatives are involved, both the D and the L forms are included, and to derivatives, especially aspartic acid β-esters or N-guanidine-acyl derivatives of arginine, to prodrugs, and also to the physiologically acceptable salts thereof.

2. An enantiomer or a diastereomer of a compound of the formula I according to Claim 1.

3. (a) Cyclo-(Arg-Gly-Asp-D-Phe-Nle);
(b) cyclo-(Arg-Gly-Asp-DPhe-hPro);
(c) cyclo-(Arg-Gly-Asp-DPhe-Tle);
(d) cyclo-(Arg-Gly-Asp-Phe-DAhds);
(e) cyclo-(Arg-Gly-Asp-Phe-Nhdg);
(f) cyclo-(Arg-Gly-Asp-DPhe-Acha),
(g) cyclo-(Arg-Gly-Asp-Dphe(4-Cl)-Tle);
(h) cyclo-(Arg-Gly-Asp-Dphe(4-F)-Tle);
according to Claim 1 and also their physiologically acceptable salts.

4. Process for the preparation of a compound of the formula I according to Claim 1 or one of its salts, **characterized in that** it is liberated from one of its functional derivatives by treatment with a solvolysing or hydrogenolysing agent
or **in that** a peptide of the formula II
H-Z-OH II,
in which
Z is -Arg-Gly-Asp-X-Y-
-Gly-Asp-X-Y-Arg-
-Asp-X-Y-Arg-Gly-
-X-Y-Arg-Gly-Asp- or
-Y-Arg-Gly-Asp-X-,
or a reactive derivative of such a peptide, is treated with a cyclizing agent,
or **in that** a cyclopeptide which corresponds per se to the formula I but which also has one or more free amino groups, acid groups and/or activated α carbon atoms is derivatized by alkylation, acylation or esterification,
and/or **in that** a basic or acidic compound of the formula I is converted into one of its salts by treatment with an acid or base.

5. Process for the production of pharmaceutical preparations, **characterized in that** a compound of the formula I according to Claim 1 and/or one of its physiologically acceptable salts is brought into an appropriate dosage form together with at least one solid, liquid or semi-liquid excipient or auxiliary.

6. Pharmaceutical preparation, **characterized by** a content of at least one compound of the general formula I according to Claim 1 and/or one of its physiologically acceptable salts.

7. Use of compounds of the formula I according to Claim 1 or of their physiologically acceptable salts for the production of a medicament for the control of diseases.

8. Use of compounds of the formula I according to Claim 1 for the production of immobilized ligands for affinity column chromatography.

9. Use of compounds of the formula I according to Claim 1 for the purification of integrins by affinity chromatography.

## Revendications

1. Composés de formule I
Cyclo-(Arg-Gly-Asp-X-Y)
où
X est Phe, homoPhe, Phg, Phe(4-F) ou Phe(4-Cl),
Y est hPro, Ahds, Aos, Nhdg, Acha, Aib, Acpa ou Tle,
y compris les formes D ainsi que L, dans la mesure où il s'agit de radicaux d'acides aminés et de dérivés d'acides aminés optiquement actifs, et les dérivés, notamment les esters β d'acide asparaginique ou les dérivés acyles de N-guanidine de l'arginine, ainsi que leurs sels physiologiquement inoffensifs.

2. Enantiomère ou diastéréomère d'un composé de formule I selon la revendication 1.

3. (a) Cyclo-(Arg-Gly-Asp-DPhe-Nle) ;
(b) Cyclo-(Arg-Gly-Asp-DPhe-hPro) ;
(c) Cycle-(Arg-Gly-Asp-DPhe-Tle) ;
(d) Cyclo-(Arg-Gly-Asp-Phe-DAhds);
(e) Cycle-(Arg-Gly-Asp-Phe-Nhdg);
(f) Cyclo-(Arg-Gly-Asp-DPhe-Acha) ;
(g) Cyclo-(Arg-Gly-Asp-Dphc(4-Cl)-Tic) ;
(h) Cyclo-(Arg-Gly-Asp-Dphe(4-F)-Tle) ;
selon la revendication 1 ainsi que leurs sels physiologiquement inoffensifs.

4. Procédé de fabrication d'un composé de formule I selon la revendication 1 ou un de ses sels, **caractérisé en ce qu'**on le libère d'un de ses dérivés fonctionnels en le traitant avec un agent solvolysant ou hydrogénolysant, ou qu'on traite avec un agent cyclisant un peptide de formule II
H-Z-OH II
où
Z est -Arg-Gly-Asp-X-Y-
-Gly-Asp-X-Y-Arg-
-Asp-X-Y-Arg-Gly-
-X-Y-Arg-Gly-Asp- ou
-Y-Arg-Gl-AspX-,
ou un dérivé d'un tel peptide susceptible de réagir, ou que l'on dérive par alkylation, acylation ou estérification un cyclopeptide qui correspond à la formule I, mais qui dispose aussi d'un ou de plusieurs groupes libres d'amines, d'acides et/ou d'atomes de C α, et/ou que l'on transforme un composé basique ou acide de formule I par traitement avec un acide ou une base en un de ses sels.

5. Procédé de fabrication de préparations pharmaceutiques, **caractérisé en ce que** l'on associe un composé de formule I selon la revendication 1 et/ou un de ses sels physiologiquement inoffensifs avec au moins un support ou un auxiliaire solide, liquide ou semi-liquide en une forme de dosage appropriée.

6. Préparation pharmaceutique, **caractérisée par** une teneur en au moins un composé de formule générale I selon la revendication 1 et/ou un de ses sels physiologiquement inoffensifs.

7. Utilisation de composés de formule I selon la revendication 1 ou de leurs sels physiologiquement inoffensifs pour fabriquer un médicament pour lutter contre les maladies.

8. Utilisation de composés de formule I selon la revendication 1 pour fabriquer des ligands immobilisés pour la chromatographic en colonnc d'affinité.

9. Utilisation de composés de formule I selon la revendication 1 pour éliminer des intégrines par chromatographie d'affinité.
